## Europäisches Patentamt
## European Patent Office
## Office européen des brevets

(19)

(11) Numéro de publication: **0 231 687**
**B1**

(12) **FASCICULE DE BREVET EUROPEEN**

(45) Date de publication du fascicule du brevet:
07.02.90

(51) Int. Cl.⁴: **A61M 1/12**

(21) Numéro de dépôt: **86402748.7**

(22) Date de dépôt: **10.12.86**

---

(54) **Prothèse cardiaque totale comportant deux modules de pompage reliés par une communication gazeuse.**

---

(30) Priorité: **16.12.85 FR 8518600**

(43) Date de publication de la demande:
**12.08.87 Bulletin 87/33**

(45) Mention de la délivrance du brevet:
**07.02.90 Bulletin 90/6**

(84) Etats contractants désignés:
**DE GB IT**

(56) Documents cités:
**EP-A- 0 146 445**
**WO-A-85/02339**
**US-A- 4 167 046**

**ARCHIVES OF SURGERY,**
**vol. 112, décembre 1977, pages 1430-1438; W.S. PIERCE et al.: "The artificial heart"**

(73) Titulaire: **AEROSPATIALE SOCIETE NATIONALE INDUSTRIELLE Société Anonyme dite:, 37, Boulevard de Montmorency, F-75016 Paris(FR)**

(72) Inventeur: **Chareire, Jean-Louis, 66, rue Aristide Briand, F-92300 Levallois(FR)**
Inventeur: **Lapeyre, Didier, Chaignes, F-27120 Pacy-Sur-Eure(FR)**

(74) Mandataire: **Bonnetat, Christian et al, CABINET BONNETAT 23, Rue de Léningrad, F-75008 Paris(FR)**

---

## Description

Des documents FR-A 8 318 368 et EP-A 0 146 445 sont connues deux modes de réalisation d'une prothèse cardiaque totale comprenant deux pompes, respectivement représentatives du coeur droit et du coeur gauche, cette prothèse étant remarquable en ce que, d'une part, elle comporte l'unité fonctionnelle indissociable constituée :

- d'un module péricardique, destiné à être logé dans la cavité du coeur naturel à remplacer et enfermé dans une enveloppe étanche portant au moins trois orifices de raccord destinés respectivement à être raccordés à l'oreillette droite, à l'artère pulmonaire et à l'oreillette gauche, lesdits orifices de raccord à l'oreillette droite et à l'artère pulmonaire étant pourvus de valves pour servir respectivement d'orifice d'entrée et d'orifice de sortie à une première pompe logée dans ledit module péricardique et destinée à accomplir la fonction du coeur droit du coeur naturel à remplacer ;

- d'un module extra-péricardique, destiné à être logé dans un espace physiologiquement neutre du malade receveur et à accomplir la fonction du coeur gauche du coeur naturel à remplacer, ce module extra-péricardique comportant une enveloppe étanche dans laquelle est enfermée une seconde pompe pourvue d'un orifice d'entrée et d'un orifice de sortie, pourvus chacun d'une valve;

- d'une liaison fonctionnelle entre lesdits modules péricardique et extra-péricardique comportant au moins :

- un premier conduit traversant l'enveloppe dudit module péricardique et réunissant l'orifice de celui-ci correspondant à l'oreillette gauche et l'orifice d'entrée de ladite seconde pompe incorporée dans ledit module extra-péricardique;

- un second conduit établissant une communication gazeuse entre les côtés desdites première et seconde pompes opposés au sang traversant celles-ci.

Dans le premier mode de réalisation, l'enveloppe étanche du module péricardique comporte un quatrième orifice destiné au raccord à l'aorte et ladite liaison fonctionnelle comporte un troisième conduit traversant l'enveloppe dudit module péricardique et réunissant ledit quatrième orifice de celui-ci correspondant à l'aorte et l'orifice de sortie de ladite seconde pompe incorporée dans ledit module extra-péricardique.

En revanche, dans le second mode de réalisation, l'orifice de sortie de ladite seconde pompe incorporée dans ledit module extra-péricardique est directement raccordé à la partie abdominale de l'aorte, de sorte que l'on peut faire l'économie du quatrième orifice du module péricardique et dudit troisième conduit de liaison fonctionnelle.

Dans ces deux modes de réalisation, on réalise avantageusement chacun desdits modules de pompage péricardique et extra-péricardique sous la forme d'une pompe à membrane et à plateau-poussoir.

La présente invention a pour objet de décrire un mode de réalisation particulièrement avantageux d'une telle prothèse cardiaque totale.

A cette fin, selon l'invention, la prothèse cardiaque totale comprenant deux pompes, respectivement représentatives du coeur droit et du coeur gauche et comportant l'unité fonctionnelle indissociable constituée :

- d'un module péricardique, destiné à être logé dans la cavité du coeur naturel à remplacer et enfermé dans une enveloppe étanche portant au moins trois orifices de raccord destinés respectivement à être raccordés à l'oreillette droite, à l'artère pulmonaire et à l'oreillette gauche, lesdits orifices de raccord à l'oreillette droite et à l'artère pulmonaire étant pourvus de valves pour servir respectivement d'orifice d'entrée et d'orifice de sortie à une première pompe du type à membrane logée dans ladite enveloppe et destinée à accomplir la fonction du coeur droit du coeur naturel à remplacer;

- d'un module extra-péricardique, destiné à être logé dans un espace physiologiquement neutre du malade receveur et à accomplir la fonction du coeur gauche du coeur naturel à remplacer, ce module extra-péricardique comportant une seconde pompe du type à membrane et plateau-poussoir pourvue d'un système d'actionnement et enfermée dans une enveloppe étanche, pourvue d'un orifice d'entrée et d'un orifice de sortie, équipés chacun d'une valve;

- d'une liaison fonctionnelle entre lesdits modules comportant au moins :

- un tube traversant ladite enveloppe dudit module péricardique et réunissant l'orifice de celui-ci correspondant à l'oreillette gauche et l'orifice d'entrée de la seconde pompe incorporée dans ledit module extra-péricardique;

- des moyens de communication gazeuse entre les côtés desdites première et seconde pompes opposés au sang traversant celle-ci;

est remarquable en ce que lesdits moyens de communication comportent :

- un premier conduit permettant d'amener à ladite première pompe le gaz déplacé par la seconde pompe, de sorte que ledit gaz déplacé actionne ladite première pompe dans le sens de l'expulsion de sang; et

- un second conduit permettant de ramener le gaz de la première pompe vers ladite seconde pompe.

Ainsi, le module péricardique ne comporte pas de système d'actionnement électromécanique, mais est actionné par la pompe du module extra-péricardique. Ceci est particulièrement avantageux puisque le module extra-péricardique peut être logé dans un espace physiologique aisément accessible par une intervention chirurgicale bénigne et peut donc être facilement changé en cas de panne du système d'actionnement des pompes. En revanche, le module péricardique étant d'accès délicat à cause de la cage thoracique, il est bon qu'il ne comporte aucun élément mécanique susceptible d'usure ou de panne.

De façon déjà décrite dans les demandes de brevets rappelées ci-dessus, il est avantageux que ladite liaison fonctionnelle réunisse la partie inférieure de l'enveloppe du module péricardique à la partie supérieure du module extra-péricardique.

Avantageusement, ledit second conduit de la liaison entre les modules péricardique et extra-péricardique est souple longitudinalement, mais rigide radialement et il enferme ledit premier conduit, ainsi que le ou les tubes de liaison sanguine entre les deux modules.

Ainsi, ledit second conduit sert de protection mécanique aux conduits ou tubes de liaison liquide et gazeuse entre lesdits modules. Ceci est particulièrement avantageux du fait que le tube reliant l'oreillette gauche et l'orifice d'entrée de la pompe du module extra-péricardique doit être très souple, donc très mince, et ne subir aucune perte de charge provoquée. Ce tube, associé à l'espace intérieur dudit second conduit, fait alors fonction d'oreillette gauche complémentaire. On remarquera que, grâce audit second conduit, ladite liaison entre les modules sert de chambre de compliance pour l'ensemble de la prothèse.

Dans ce second conduit, on peut faire passer un câble électrique reliant lesdits modules péricardique et extra-péricardique. Ainsi, la commande des valves dudit module pédicardique peut passer par ledit module extra-péricardique et ledit câble électrique contenu dans la liaison.

Dans un mode avantageux de réalisation, ladite première pompe est du type à membrane et plateau-poussoir, et du côté opposé à la circulation sanguine du coeur droit, ceux-ci délimitent avec un boîtier une chambre étanche à laquelle sont reliés, par l'intermédiaire de soupapes électro-commandées, lesdits premier et second conduits.

Ledit premier conduit est solidaire de façon étanche dudit boîtier, tandis que ledit second conduit débouche dans une cavité de l'enveloppe du module péricardique en liaison avec ladite chambre par l'intermédiaire d'une desdites soupapes.

Ainsi, le ou les tubes de liaison sanguine entre les modules péricardique et extra-péricardique peuvent passer à travers ladite cavité.

De préférence, ladite seconde pompe associée au module extra-péricardique comporte, dans son enveloppe, d'une part, une enceinte étanche en communication avec ledit orifice d'entrée et ledit orifice de sortie de ladite seconde pompe et délimitée au moins en partie par deux membranes souples en regard et, d'autre part, deux plateaux-poussoirs en regard l'un de l'autre, disposés de part et d'autre de ladite enceinte étanche au contact d'une membrane et animés de mouvements alternatifs d'éloignement et de rapprochement, sous l'action de mécanismes agencés respectivement du côté du plateau-poussoir correspondant opposé à ladite enceinte étanche.

Dans ce cas, lesdits premier et second conduits des moyens de communication sont en communication avec les parties internes de ladite enveloppe extérieures à ladite enceinte étanche et contenant lesdits mécanismes d'actionnement.

On remarquera qu'ainsi, ladite seconde pompe est constituée de deux systèmes de pompages agissant sur la même enceinte étanche déformable, de sorte que, même si l'un desdits systèmes tombe en panne, le pompage peut continuer sous l'action de l'autre.

De façon générale, le module extra-péricardique de la prothèse selon l'invention peut présenter l'une ou l'autre des structures qui forment le sujet de la demande européene 86 402 746.1 publiée comme EP-A 227 537 le 01.07.87.

Les figures du dessin annexé feront bien comprendre comment l'invention peut être réalisée. Sur ces figures, des références identiques désignent des éléments semblables.

La figure 1 montre schématiquement un coeur naturel en liaison avec ses veines et artères principales, en vue antérieure.

La figure 2 illustre schématiquement une prothèse conforme à l'invention, en place sur un patient.

La figure 3 est une vue extérieure schématique de face d'un mode de réalisation du module péricardique selon l'invention, pour la prothèse de la figure 2.

La figure 4 est une vue schématique, en coupe partielle suivant un plan orthogonal au plan de la figure 3, du module péricardique représenté sur cette dernière figure.

La figure 5 est une vue extérieure, en perspective éclatée, d'un mode de réalisation du module extra-péricardique selon l'invention, pour la prothèse de la figure 2.

La figure 6 est une vue en élévation latérale du module extra-péricardique de la figure 5.

Les figures 7 et 8 sont respectivement des vues de côté et du dessus du module extra-péricardique de la figure 5.

La figure 9 est une coupe selon la ligne IX-IX de la figure 7.

La figure 10 montre schématiquement en coupe un mode de réalisation du mécanisme d'actionnement du module extra-péricardique des figures 5 à 9.

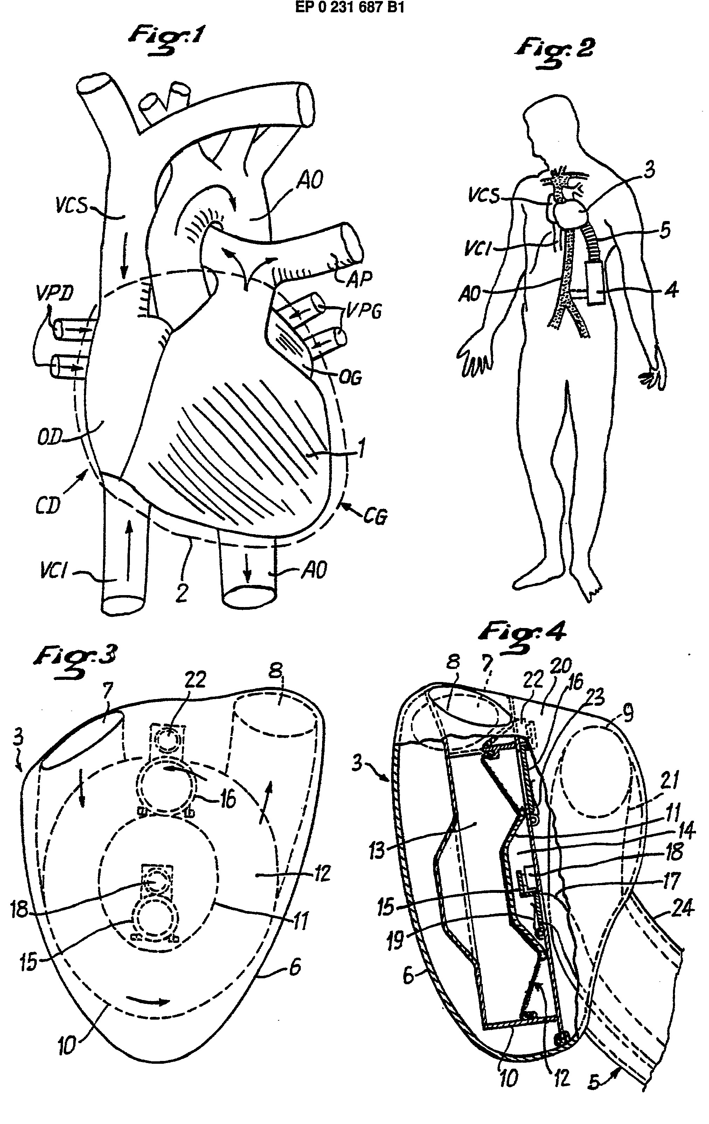

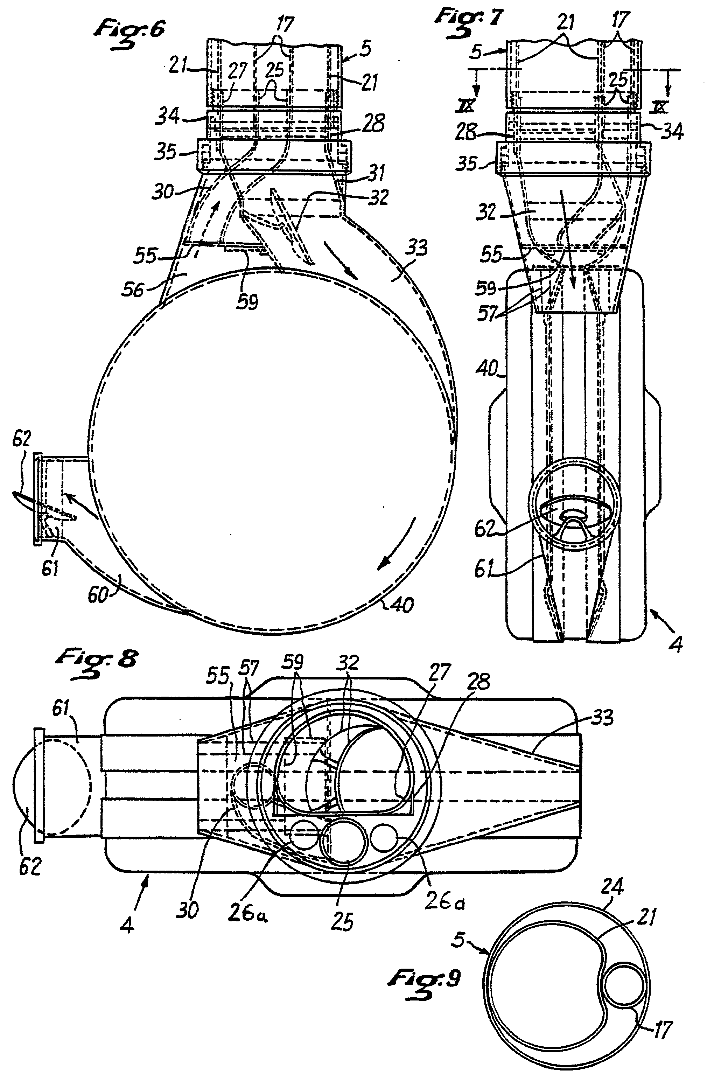

L'exemple de réalisation de la prothèse selon l'invention, représentée sur les figures 2 à 9 et choisie pour illustrer la présente invention, concerne plus particulièrement un mode de réalisation dans lequel le module extra-péricardique est directement relié à l'aorte abdominale. Cependant, cette disposition n'est pas obligatoire et la présente invention s'applique tout autant à une prothèse dans laquelle le module péricardique est relié à l'aorte thoracique, une liaison sanguine supplémentaire étant alors prévue entre les modules péricardique et extra-péricardique.

Comme illustré schématiquement sur la figure 1, un coeur humain naturel 1 est logé dans la cavité péricardique 2 (simplement illustrée par un trait tireté 2) et est composé en réalité de deux coeurs distincts, mais solidaires l'un de l'autre, à savoir le coeur droit CD comportant l'oreillette droite OD et le ventricule droit et le coeur gauche CG comportant l'oreillette gauche OG et le ventricule gauche. L'oreillette OD du coeur droit CD reçoit le sang veineux par la veine cave supérieure VCS et par la veinte cave inférieure VCI, tandis que le ventricule dudit coeur droit CD fait passer le sang ainsi reçu vers les poumons par l'intermédiaire de l'artère pulmonaire AP.

De même, l'oreillette OG du coeur gauche CG reçoit le sang provenant des poumons par les veines pulmonaires gauches VPG et droites VPD et le ventricule du coeur gauche CG chasse le sang reçu par l'aorte AO.

L'idée de base de la prothèse à pompes découplées du type auquel se rapporte la présente invention repose sur la constatation physiologique, que, quoique constitué de deux pompes CD et CG formant une unité musculaire unique, le coeur 1 est en réalité composé de deux ensembles fonctionnellement indépendants. En effet, sur le plan fonctionnel, le coeur droit CD peut être considéré comme un simple coeur de passage qui pousse une colonne sanguine dont la vitesse d'écoulement est variable, mais jamais nulle, sauf lorsque les fréquences des battements du coeur 1 sont très basses. Lorsque le débit sanguin du système vasculaire augmente par suite d'une augmentation de fréquence de ces battements, la participation du coeur droit CD à la mise en mouvement du sang dans le circuit pulmonaire diminue du fait de l'augmentation de la vitesse, et donc de l'énergie cinétique, du sang parvenant au coeur droit CD. En revanche, le coeur gauche CG, par son puissant ventricule, constitue le coeur proprement dit, c'est-à-dire la pompe propulsive chargée d'assurer la perfusion sanguine de tous les organes et tissus de l'organisme.

Comme l'illustre très schématiquement la figure 2, cette prothèse cardiaque totale est constituée d'une unité fonctionnellement indissociable constituée de deux modules de pompage 3 et 4 découplés, mais reliés l'un à l'autre par une liaison fonctionnelle tubulaire 5.

Le module de pompage 3, destiné à remplacer le-coeur droit CD du coeur naturel 1, est logé dans la cavité péricardique 2. Il comporte une enveloppe étanche sur laquelle sont montés des embouts de raccord de tout type connu, respectivement destinés à le raccorder à l'oreillette droite OD (réservoir des veines caves VCI et VCS), à l'artère pulmonaire AP, à l'oreillette gauche OG (réservoir des veines pulmonaires VPG et VPD) et, éventuellement, à l'aorte AO, après coupe de celles-ci et exérèse des ventricules naturels de la cavité péricardique 2.

Les orifices de raccord à l'oreillette droite OD et à l'artère pulmonaire AP sont pourvus de valves pour servir respectivement d'orifice d'entrée et d'orifice de sortie à une pompe logée dans ladite enveloppe et destinée à accomplir la fonction du coeur droit du coeur naturel à remplacer.

Le module de pompage 4, destiné à joeur le rôle du coeur gauche CG du coeur naturel 1, est logé à l'extérieur de la cavité péricardique 2, dans un espace physiologiquement neutre, par exemple du thorax ou de l'abdomen. Il comporte également une enveloppe étanche enfermant une pompe pourvue d'un orifice d'entrée et d'un orifice de sortie, équipés chacun d'une valve.

La liaison fonctionnelle 5, qui peut sans inconvénient traverser le diaphragme du patient recevant la prothèse, comporte :

- un conduit assurant la connexion entre l'orifice du module péricardique 3 correspondant à l'oreillette gauche OG et l'orifice d'entrée de la pompe incorporée dans le module extra-péricardique 4;

- éventuellement, lorsque l'orifice de sortie de la pompe du module extra-péricardique n'est pas relié directement à l'aorte abdominale, un conduit assurant la connexion entre l'orifice du module péricardique 3 correspondant à l'aorte AO et l'orifice de sortie de ladite pompe incorporée dans ledit module extra-péricardique 4;

- une communication gazeuse entre les côtés des pompes du module péricardique et du module extra-péricardique opposés au sang traversant celles-ci.

Sur la figue 2, on a représenté schématiquement en pointillés le fait que l'orifice de sortie de la pompe du module extra-péricardique 4 pouvait être directement relié à la partie abdominale de l'aorte. Dans ce cas, le module péricardique 3 ne comporte pas d'orifice de raccord à l'aorte.

Le module péricardique 3 de la prothèse est disposé dans une partie du corps humain qui n'est accessible que par une opération chirurgicale délicate; cependant, le travail de pompage qu'il effectue est peu important. En revanche, le module extra-péricardique 4 de ladite prothèse est disposé dans une partie du corps humain facilement accessible au prix d'une bénigne intervention chirurgicale; mais le travail de pompage qu'il effectue est important.

Par ailleurs, il résulte, du fait que le volume dans lequel se trouve le module extra-péricardique est important, que celui-ci peut être construit sans que l'on ait à tenir compte de contraintes d'encombrement trop critiques. Le mécanisme d'actionnement du module extra-péricardique peut donc être puissant.

Dans ces conditions, l'idée de base de l'invention est d'utiliser ce puissant mécanisme extre-péricardique pour actionner la pompe péricardique qui, ainsi, peut ne comporter qu'un minimum d'éléments sujets à usure et à défaillance.

Le mode de réalisation du module péricardique 3 montré par les figures 3 et 4 comporte une enveloppe étanche 6 pourvue d'orifices 7,8 et 9 (représentés schématiquement) destinés à être raccordés respectivement à l'oreillette droite, à l'artère pulmonaire et à la veine pulmonaire du coeur défaillant à remplacer.

A l'intérieur de l'enveloppe étanche 6 est disposé un boîtier étanche 10 enfermant un plateau rigide central de pompage 11 appliqué contre au moins une membrane souple 12. L'intérieur du boîtier étanche 10 est partagé en deux chambres étanches 13 et 14 par la membrane 12. La chambre étanche 13 est en liaison avec les orifices 7 et 8 par des soupapes non représentées, tandis que la chambre étanche 14 est pourvue de deux soupages électrocommandées 15 et 16.

La soupage 15 est reliée de façon étanche avec un conduit 17 faisant partie de la liaison 5 et destiné à véhiculer le gaz mis en pression par la pompe du module extra-péricardique 4, comme on le verra ci-après. La soupage 15 est commandée par un électroaimant 18 et est soumise à l'action d'un ressort de rappel 19.

La soupage 16 fait communiquer la chambre 14 avec une cavité 20 de l'enveloppe 6 à travers laquelle passe la veine pulmonaire artificielle 21 reliant les deux modules 3 et 4. La soupage 16 est commandée par un électro-aimant 22 est est soumise à l'action d'un ressort de rappel 23.

La cavité 20 de l'enveloppe 6 est en liaison gazeuse avec le module 4 (comme on le verra ci-après) par un conduit 24, rigide radialement, mais souple en flexion.

Le conduit 24 sert d'enveloppe extérieure pour la liaison 5 et il enferme le conduit 17 et la veine pulmonaire artificielle 21. Si le module péricardique 3 était destiné à être relié à l'aorte, le conduit 24 enfermerait de plus une aorte artificielle reliant les deux modules 3 et 4.

Lorsque le gaz déplacé par la pompe du module extra-péricardique 4 arrive par le conduit 17 dans le module péricardique 3, il se comprime du fait que la soupape 15 est fermée. Lorsque la pression de gaz atteint une valeur suffisante pour être en mesure de fournir au sang la courbe de pression physiologiquement désirée, la soupape 15 s'ouvre car le courant est coupé dans l'électro-aimant 18. Le gaz pénètre dans la chambre 14 et agit sur le plateau poussoir rigide 11 relié à la membrane souple 12.

Lorsque le volume de sang expulsé par la pompe péricardique 10,11 est suffisant, la soupage 16 s'ouvre, car on coupe le courant de l'électro-aimant 22 et son ressort de rappel 23 ne s'oppose pas au retour du gaz vers le module 4. Pendant ce temps, le ressort de rappel 19 referme la soupage 15 et le courant est rétabli dans l'électro-aimant 18.

Ce retour de gaz s'effectue à travers la partie du conduit 24 non occupée par les conduits 17 et 21, ce qui permet la mise au contact permanente avec la pression atmosphérique par l'intermédiaire de la pression veineuse qui règne dans le tube souple et extensible 21.

La réalisation du module péricardique 3 ne pose aucun problème particulier.

L'enveloppe 6 peut être réalisée en un polycarbonate injecté ou en un alliage métallique biocompatible,électro-déposé par exemple. Le boîtier 10 peut être réalisé par emboutissage et soudage ou tout autre procédé. L'intérieur de ce boîtier doit être hémocompatible. On peut donc le revêtir de carbone déposé par le procédé connu de dépôt physique en phase vapeur ou de polyuréthane déposé par trempage (par exemple). Le plateau-poussoir rigide 11 peut être métallique ou en plastiques divers, car il est toujours revêtu d'une membrane hémocompatible.

Les soupapes sont par exemple métalliques (titane, acier inoxydable etc...). Par exemple, le tube 21 est en polyuréthane et le tube 17 en élastomère. Le tube 24 peut être en une matière synthétique renforcée d'une hélice de fil d'acier.

On voit sur les figures 3 et 4 que le débit sanguin n'est jamais interrompu dans le module 3 grâce à la position de la membrane. Ainsi comme on le voit sur la figure 3, il y aura toujours un tourbillon sanguin dans le même sens (indiqué par les flèches). Cette disposition permet d'avoir un bon rendement hydraulique et de lutter efficacement contre les tendances à la thrombose.

Par ailleurs, la membrane peut être munie de détecteurs de fuites. En cas de détection de fuite, l'alarme est donnée et, en cas de détection de fuite grave, il y a mise hors service totale du module 3 en maintenant en permanence les valves 15 et 16 fer-mées. Le sang ne pourra donc pas envahir la zone des moteurs du module extra-péricardique 4. Le malade survivra à cette panne extrême, car la pression de retour veineux est suffisante pour assurer la traversée du ventricule droit et des poumons jusqu'au moment de l'intervention chirurgicale.

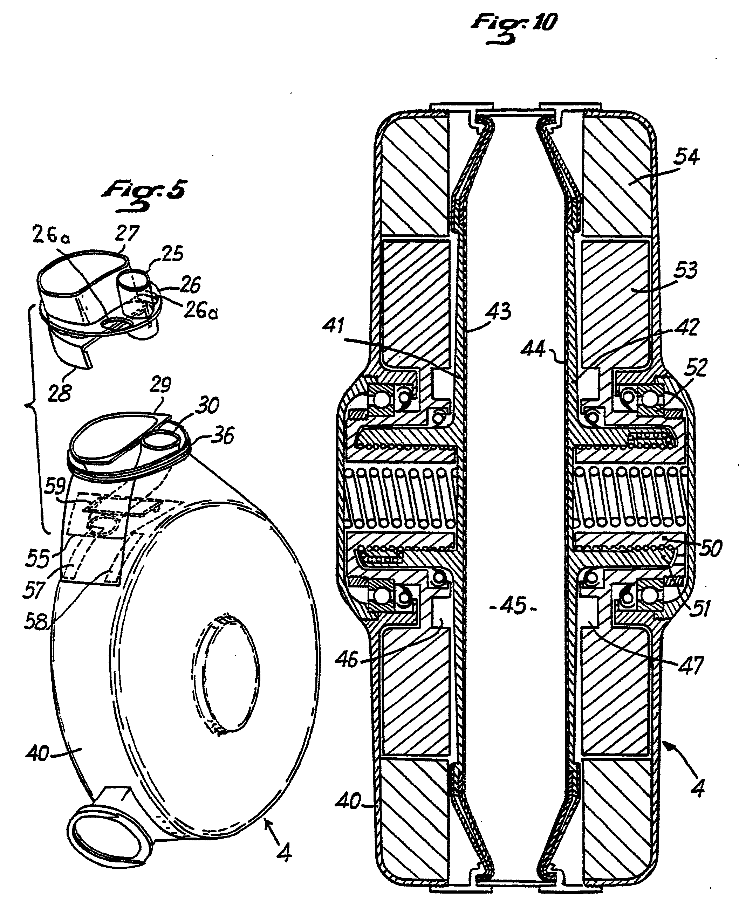

Sur les figures 5 à 10, on a représenté un mode de réalisation du module extra-péricardique 4 susceptible de coopérer avec le module péricardique 3 décrit ci-dessus. Ce module 4 comporte une enveloppe 40 à l'intérieur de laquelle est enfermée une pompe uniquement montrée sur la figure 10 et qui est reliée par la liaison 5 au module 3.

Le conduit souple 17 du gaz comprimé est raccordé sur um embout métallique 25. Cet embout 25 est soudé sur une plaque métallique 26 portant un autre embout 17 auquel est raccordé le tube 21.

Tout cet ensemble 25,26 et 27 est soudé sur une pièce de raccord 28 qui vient plaquer, en position de montage définitif, sa face intérieure contre une plaque 29. Dans cette position, le tube 25 coïncide avec un tube rigide 30 dont l'extrémité supérieure est dans le même plan que la surface de la plaque 29. Le tube 30, la plaque 29 et un tuyau rigide 31 qui prolonge le tube 27 sont soudés ensemble et rejoignent par des liaisons rigides une soupage 32 et une volute 33 d'introduction du sang dans le module extra-péricardique 4.

La pièce 34 de fixation du tube 24 vient prendre appui sur un joint qui exerce une force axiale sur la pièce 26 qui permet d'avoir l'étanchéité entre les tubes 25 et 30 et entre les pièces 28 et 29. La surface de la pièce 29 est recouverte d'une très fine couche organique molle pour favoriser le glissement et l'étanchéité lors du montage. Un écrou de serrage 35 vient se visser sur un col fileté 36 solidaire de l'enveloppe 40.

Comme le montre la figure 10, qui donne un exemple de réalisation du mécanisme de pompage du module 4, ce mécanisme comporte deux plateaux-poussoirs 41 et 42 en vis-à-vis pouvant se rapprocher et s'écarter l'un de l'autre et prenant appui sur des membranes souples 43 et 44 respectivement.

A chacun des plateaux-poussoirs 41 et 42 est associé une vis 50 et un écrou 51 à circulation de billes, solidaire du plateau-poussoir correspondant.

La vis 50 tourne sur son axe grâce à un roulement à billes à gorge profonde 52 qui permet un repérage géométrique suffisant. La vis 50 est reliée au rotor 53 d'un moteur électrique plat dont le stator annulaire porte la référence 54.

L'obtention du déplacement axial d'un plateau 41,42 s'obtient par rotation du rotor 53 qui fait tourner la vis 50, le plateau étant immobilisé en rotation par les membranes 43 et 44.

A l'intérieur de l'enveloppe 40, les deux membranes souples 43 et 44 délimitent entre elles, une enceinte étanche 45 en liaison avec le conduit (veine pulmonaire artificielle) 21, de la façon décrite ci-dessus. De plus, avec l'enveloppe 40, les deux membranes souples 43 et 44 délimitent deux espaces 46 et 47 extérieurs à l'enceinte étanche 45. Les espaces 46 et 47 sont en liaison avec les conduits 17 et 24 de la manière qui sera décrite ci-après.

Le gaz se dirigeant du module extra-péricardique vers le module péricardique passe par le tuyau rigide 30 qui est soudé de manière étanche à une plaque 55 délimitant un volume intérieur 56 qui communique avec les espaces gazeux 46 et 47 situés dans le module extra-péricardique 4 du côté des plateaux 41 et 42 opposé au sang et comprenant par conséquent les moteurs électriques. La communication avec chacun des deux volumes concernés s'effectue par des orifices 57 et 58 respectivement.

Le gaz qui revient du module péricardique 3 sous faible pression passe par des trous 26a de la pièce 26. Il circule ensuite entre les pièces 30 et 31 et l'enveloppe externe locale du module extra-péricardique jusqu'à la plaque 55. Il rencontre alors une soupape 59 qui permet le passage (en retour seulement) à travers la plaque 55. Il passe ensuite dans les orifices 57 et 58.

Comme on peut le voir sur la figure 9, le tuyau très souple et extensible 21 se répand librement entre les formes souples mais plus rigides des conduits 24 et 17. Le volume interne résiduel entre les conduits 21, 24 et 17 sert au retour de l'air. Il dépend d'une part de la pression sanguine dans la veine pulmonaire 21 très influencée par la pression atmosphérique à la suite du passage du sang dans les poumons et d'autre part de l'ouverture ou de la fermeture de la soupape 32 puisque l'extensibilité du tuyau 21 lui permet de jouer le rôle d'oreillette artificielle complémentaire (ce qui régularise le débit pulmonaire et favorise le remplissage du ventricule gauche).

La sortie du sang vers l'aorte s'effectue par une volute 60 (en liaison avec la cavité 45) et un orifice de raccord 61 pourvu d'une soupape 62.

Ainsi, on voit que selon l'invention, la pompe du module péricardique peut être actionnée par le gaz déplacé par la pompe extra-péricardique. Il en résulte les avantages suivants :

- suppression du moteur d'activation du module péricardique et donc gain de masse et de volume pour ce module;
- augmentation de la fiabilité, car la probabilité de pannes pour l'ensemble des deux modules est à peine supérieure à celle du module extra-péricardique seul (et l'ensemble des pannes est généralement sans conséquences graves grâce à la redondance mécanique);
- possibilité d'augmentation progressive du débit du module péricardique après l'opération, puisqu'on peut ouvrir la soupape d'échappement 16 avant que la course totale des membranes de la pompe extra-péricardique soit effectuée;
-diminution des bruits et vibrations dans le péricarde;
- bonne résistance de l'intérieur du module péricardique à la thrombose grâce à la possibilité d'un tourbillon sanguin permanent;
- bon rendement hydraulique du module péricardique;
- faible fatigue des membranes du module péricardique et sécurité en cas de détérioration de celles-ci;
-économie sur le prix de revient du module péricardique;

-le fait d'actionner le module péricardique par un gaz ne pénalise que très peu le rendement global car la puissance nécessaire à l'actionnement de ce module est très inférieure à celle que nécessite le module extra-péricardique.

**Revendications**

1 - Prothèse cardiaque totale comprenant deux pompes, respectivement représentatives du coeur droit et du coeur gauche constituée :
- d'un module péricardique (3) destiné à être logé dans la cavité (2) du coeur naturel à remplacer et enfermé dans une enveloppe étanche (6) portant au moins trois orifices de raccord (7,8,9) destinés respectivement à être raccordés à l'oreillette droite OD, à l'artère pulmonaire AP et à l'oreillette gauche OG, lesdits orifices (7) et (8) de raccord à l'oreillette droite OD et à l'artère pulmonaire AP étant pourvus de valves pour servir respectivement d'orifice d'entrée et d'orifice de sortie à une première pompe (10,11) du type à membrane logée dans ladite enveloppe (6) et destinée à accomplir la fonction du coeur droit du coeur naturel à remplacer ;
- d'un module extra-péricardique (4), destiné à être logé dans un espace physiologiquement neutre du malade receveur et à accomplir la fonction du coeur gauche du coeur naturel (1) à remplacer, ce module extra-péricardique comportant une seconde pompe (41 à 44) du type à membrane et à plateau-poussoir pourvue d'un système d'actionnement (50 à 54), et enfermée dans une enveloppe étanche (40) pourvue d'un orifice d'entrée (33) et d'un orifice de sortie (61) équipés chacun d'une valve ;
- d'une liaison fonctionnelle (5) entre lesdits modules comportant au moins :
- un tube (21) traversant ladite enveloppe (6) dudit module péricardique (3) et réunissant l'orifice (9) de celui-ci correspondant à l'oreillette gauche OG et l'orifice d'entrée (33) de la seconde pompe (41 à 44) incorporée dans ledit module extra-péricardique (4);
- des moyens de communication gazeuse entre les côtés desdites première et seconde pompes opposés au sang traversant celles-ci;
caractérisée en ce que lesdits moyens de communication comportent :
- un premier conduit (17) permettant d'amener à ladite première pompe le gaz déplacé par la seconde pompe, de sorte que ledit gaz déplacé actionne ladite première pompe dans le sens de l'expulsion de sang; et
- un second conduit (24) permettant de ramener le gaz de la première pompe vers ladite seconde pompe.

2 - Prothèse cardiaque selon la revendication 1, caractérisée en ce que ledit second conduit (24) de la liaison (5) entre les modules péricardique et extra-péricardique est souple longitudinalement, mais rigide radialement et il enferme ledit premier conduit (17), ainsi que le ou les tubes de liaison sanguine (21) entre les deux modules.

3 - Prothèse cardiaque selon l'une des revendications 1 ou 2, caractérisée en ce que la première pompe est du type à membrane et plateau-poussoir et, du côté oppo-

sé à la circulation sanguine du coeur droit, ceux-ci délimitent avec un boîtier (10) une chambre étanche (14) à laquelle sont reliés, par l'intermédiaire de soupapes électrocommandées (15,16), lesdits premier et second conduits.

4 - Prothèse cardiaque selon la revendication 3, caractérisée en ce que ledit premier conduit (17) est solidaire de façon étanche dudit boîtier (10),tandis que ledit second conduit (21) débouche dans une cavité (20) de l'enveloppe du module péricardique en liaison avec ladite chambre par l'intermédiaire d'une desdites soupapes (16).

5 - Prothèse cardiaque selonl'une des revendications 1 à 4, caractérisée en ce que ladite seconde pompe associée au module extra-péricardique comporte, dans son enveloppe (40),d'une part, une enceinte étanche (45) en communication avec ledit orifice d'entrée (33) et ledit orifice de sortie (61) de ladite seconde pompe et délimitée au moins en partie par deux membranes souples en regard (43,44) et, d'autre part, deux plateaux-poussoirs (41,42) en regard l'un de l'autre, disposés de part et d'autre de ladite enceinte étanche (45) au contact d'une membrane et animés de mouvements alternatifs d'éloignement et de rapprochement, sous l'action de mécanismes agencés respectivement du côté du plateau-poussoir correspondant opposé à ladite enceinte étanche.

6 - Prothèse cardiaque selon la revendication 5, caractérisé en ce que lesdits premier et second conduits (17,21) des moyens de communication sont en communication avec les parties internes (46,47) de ladite enveloppe (40) extérieures à ladite enceinte étanche (45) et contenant lesdits mécanismes d'actionnement.

**Patentansprüche**

1. Künstliches Herz mit zwei jeweils der rechten und der linken Herzkammer entsprechenden Pumpen, bestehend aus:
– einem Herzbeutelmodul (3) zur Aufnahme im Hohlraum (2) des natürlichen, zu ersetzenden Herzens und in einer dichten, mit wenigstens drei Anschlußöffnungen (7, 8, 9) versehenen Umhüllung (6) eingeschlossen, die zum Anschluß an den rechten Herzvorhof (OD), an die Lungenarterie (AP) bzw. an den linken Herzvorhof vorgesehen sind, wobei die Öffnungen (7) und (8) zum Anschluß an den rechten Herzvorhof OD und an die Lungenarterie AP mit Ventilen versehen sind, um jeweils als Eingangs- oder als Ausgangsöffnung für eine erste Pumpe (10, 11) des Membrantyps zu dienen, die in der Umhüllung (6) untergebracht ist und zum Ausführen der Funktion der rechten Herzkammer des zu ersetzenden natürlichen Herzens vorgesehen;
– einem außerhalb des Herzbereichs angeordneten Modul (4) zur Aufnahme in einem physiologisch neutralen Körperbereich des kranken Empfängers und zum Ausführen der Funktion der linken Herzkammer des zu ersetzenden natürlichen Herzens (1), wobei dieser außerhalb des Herzbereichs angeordnete Modul eine zweite Pumpe (41 bis 44) des Membran- sowie Druckplattentyps aufweist, die mit einem Betätigungssystem (50 bis 54) versehen und in einer dichten, mit einer Eingangs- (33) und einer Ausgangsöffnung (61) versehenen Umhüllung (40) eingeschlossen ist, wobei jede der Öffnungen mit einem Ventil versehen ist;
– einer Funktionsverbindung (5) zwischen diesen Modulen mit mindestens:
– einem Rohr (21) durch die Umhüllung (6) des Herzbeutelmoduls (3), das dessen dem linken Herzvorhof OG entsprechende Öffnung (9) und die Eingangsöffnung (33) der zweiten, in dem außerhalb des Herzbereichs angeordneten Modul (4) befindliche Pumpe (41 bis 44) verbindet;
– Mitteln zur Gasverbindung zwischen den zum Blutfluß entgegengesetzten Seiten der ersten und zweiten Pumpe;
dadurch gekennzeichnet, daß die Mittel zur Gasverbindung versehen sind mit:
– einer ersten Leitung (17), die die Zufuhr des von der zweiten Pumpe verdrängten Gases zur ersten Pumpe derart erlaubt, daß das verdrängte Gas die erste Pumpe in Austreibrichtung des Bluts betätigt; und
– einer zweiten Leitung (24), die die Rückführung des Gases der ersten Pumpe zur zweiten Pumpe erlaubt.

2. Künstliches Herz nach Anspruch 1, dadurch gekennzeichnet, daß die zweite Leitung (24) der Verbindung (5) zwischen dem Herzbeutelmodul und dem außerhalb des Herzbereichs angeordneten Modul in Längsrichtung nachgiebig ist, radial jedoch starr, und daß sie die erste Leitung (17) ebenso wie die Blutverbindung oder -verbindungen (21) zwischen den beiden Modulen umgibt.

3. Künstliches Herz nach einem der Ansprüche 1 oder 2, dadurch gekennzeichnet, daß die erste Pumpe vom Membran- sowie Druckplattentyp ist, und daß auf der dem Blutfluß der rechten Herzkammer entgegengesetzten Seite mittels eines Gehäuses (10) eine dichte Kammer (14) abeteilt ist, mit der die erste und zweite Leitung über Magnetventile (15, 16) verbunden sind.

4. Künstliches Herz nach Anspruch 3, dadurch gekennzeichnet, daß die erste Leitung (17) in abdichtender Weise fest mit dem Gehäuse (10) verbunden ist, während die zweite Leitung (21) in einen Hohlraum (20) der Umhüllung des Herzbeutelmoduls mündet, der über eines der Ventile (16) mit der Kammer (14) in Verbindung steht.

5. Künstliches Herz nach einem der Ansprüche 1 bis 4, dadurch gekennzeichnet, daß die dem außerhalb des Herzbereichs angeordnete Modul zugeordnete zweite Pumpe in ihrer Umhüllung (40) einerseits eine dichte Einfassung (45) in Verbindung mit der Eingangsöffnung (33) und der Ausgangsöffnung (61) der zweiten Pumpe sowie wenigstens teilweise durch zwei elastische Membranen (43, 44) sowie andererseits zwei gegenüberstehende Druckplatten (41, 42) aufweist, die beiderseits der dichten Einfassung (45) in Kontakt mit einer Membrane angeordnet sind und die von wechselnden Hin- und Herbewegungen unter Wirkung von jeweils neben der betreffenden, der dichten Einfassung entgegengesetzten Druckplatte angeordneten Mitteln betätigt werden.

6. Künstliches Herz nach Anspruch 5, dadurch
gekennzeichnet, daß die erste und die zweite Leitung (17, 21) der Verbindungsmittel in Verbindung mit
den inneren Teilen (46, 47) der Umhüllung (40) stehen, die außerhalb der dichten Einfassung angeordnet sind und die genannten Betätigungsmittel enthal-
ten.

**Claims**

1. Complete artificial heart including two pumps,
representative respectively of the right heart and
of the left heart formed:
   - by a pericardial module (3) intended to be
   housed in the cavity (2) of the natural heart to be
   replaced and enclosed in a sealed envelope (6)
   having at least three orifices (7, 8, 9) for connection respectively to the right auricle OD, to the
   pulmonary artery AP and to the left auricle OG,
   said orifices (7) and (8) for connection to the
   right auricle OD and to the pulmonary artery AP
   being provided with valves fer serving respectively as inlet port and outlet port to a first pump
   (10, 11) of the membrane type housed in said envelope (6) and intended to provide the function of
   the right heart of the natural heart to be replaced;
   - by an extra-pericardial module (4) intended to
   be housed in a physiologically neutral space of
   the receiving patient and to provide the function
   of the left heart of the natural heart (1) to be replaced, this extra-pericardial module including a
   second pump (4, to 44) of the membrane and
   pusher plate type having an actuation system (50
   to 54) and being enclosed in a sealed envelope
   (40) provided with an inlet orifice (33) and an outlet orifice (61) each equipped with a valve;
   - by a functional connection (5) between said
   modules including at least;
   - a tube (21) passing through said envelope (6) of
   said pericardial module (3) and connecting the orifice (9) thereof corresponding to the left auricle
   OG with the inlet port (33) of the second pump (41
   to 44) incorporated in said extra-pericardial module (4) ;
   - gas communication means between the sides of
   said first and second pumps opposed to the blood
   passing therethrough, characterized in that said
   communication means include :
   - a first duct (17) for bringing to said first pump
   the gas displaced by the second pump, so that
   said displaced gas actuates said first pump in the
   blood expulsion direction; and
   - a second duct (24) for bringing the gas from the
   first pump to said second pump.

2. Artificial heart according to claim, characterized in that said second duct (24) of said connection
(5) between the pericardial and extra-pericardial
modules is flexible in the longitudinal direction, but
rigid radially, and it encloses said first duct (17) as
well as the blood connection tube or tubes (21) between the two modules.

3. Artificial heart according to one of claims 1 to
2, characterized in that the first pump is of the membrane and pusher plate type and, on the side opposed to the blood flow of the right heart, they de-
fine with a case (10) a sealed chamber (14) to which
said first and second ducts are connected through
electrocontrolled valves (15, 16).

4. Artificial heart according to claim 3, charac-
ized in that said first duct (17) is sealingly secured
to said case (10), whereas said second duct (21)
opens into a cavity (20) of the envelope of the pericardial module in connection with said chamber
through one of said valves (16).

5. Artificial heart to one of claims 1 to 4, characterized in that said second pump associated with the
extra-pericardial module includes, in its envelope
(40), on the one hand, a sealed enclosure (45) in
communication with said inlet port (33) and said outlet port (61) of said second pump and defined at
least partially by two flexible facing membranes (43,
44) and, on the other hand, two pusher plates (41,
42) facing each other, disposed on each side of
said sealed enclosure (45) in contact with a membrane and driven with a reciprocal movement mov-
ing them together and away from each other, under
the action of mechanisms arranged respectively on
the side of the corresponding pusher plate opposite
said sealed enclosure.

6. Artificial heart according to claim 5, characterized in that said first and second ducts (17, 21) of
said communication means are in communication with
the internal parts (46, 47) of said envelope (40) ex-
ternal to said sealed enclosure (45) and containing
said actuation mechanisms.

Fig.1

Fig.2

Fig.3

Fig.4

Fig. 10

Fig. 5

*Fig.6*

*Fig.7*

*Fig.8*

*Fig.9*